# EUROPEAN PATENT SPECIFICATION

(11) **EP 2 411 815 B1**
(45) Date of publication and mention of the grant of the patent: **11.11.2015**
(21) Application number: 10709742.0
(22) Date of filing: 23.03.2010
(51) Int. Cl.: G01N 33/68, G01N 33/50, G01N 33/574

(54) **BIOMARKER FOR MONITORING PATIENTS**
BIOMARKER ZUM MONITORING VON PATIENTEN
BIOMARQUEUR POUR SURVEILLER DES PATIENTS

(30) Priority: 24.03.2009 EP 09305256
(43) Date of publication of application: 01.02.2012
(73) Proprietor: Transgene SA, 67405 Illkirch Graffenstaden Cedex (FR)
(72) Inventor: ACRES, Bruce, 67000 Strasbourg (FR); MARIE-BASTIEN, Bérangère, 67230 Huttenheim (FR)
(74) Representative: Vossius & Partner Patentanwälte Rechtsanwälte mbB
(86) International application number: PCT/EP2010/053755
(87) International publication number: WO 2010/108908

(56) References cited:
- EP-A1- 0 958 826
- WO-A2-98/37095
- WO-A2-02/076485
- WO-A2-2007/015171
- MARAVEYAS A ET AL: "Possible improved survival of patients with stage IV AJCC melanoma receiving SRL 172 immunotherapy: correlation with induction of increased levels of intracellular interleukin-2 in peripheral blood lymphocytes." ANNALS OF ONCOLOGY : OFFICIAL JOURNAL OF THE EUROPEAN SOCIETY FOR MEDICAL ONCOLOGY / ESMO JUL 1999 LNKD- PUBMED:10470429, vol. 10, no. 7, July 1999 (1999-07), pages 817-824, XP002583336 ISSN: 0923-7534
- NOBIRON ISABELLE ET AL: "Co-administration of IL-2 enhances antigen-specific immune responses following vaccination with DNA encoding the glycoprotein E2 of bovine viral diarrhoea virus" VETERINARY MICROBIOLOGY, vol. 76, no. 2, 25 September 2000 (2000-09-25), pages 129-142, XP002583337 ISSN: 0378-1135
- NEWMAN KIMBERLEY D ET AL: "Delivery of MUC1 mucin peptide by poly(d,l-lactic-co-glycolic acid) microspheres induces type 1 T helper immune responses" JOURNAL OF PHARMACEUTICAL SCIENCES, vol. 87, no. 11, November 1998 (1998-11), pages 1421-1427, XP002583338 ISSN: 0022-3549
- ACRES BRUCE ET AL: "A signature of circulating biomarkers correlates with clinical outcome in a randomized phase IIb trial evaluating the therapeutic vaccine TG4010 in NSCLC patients." POSTER, 19 April 2009 (2009-04-19), XP002583339 & 100TH ANNUAL MEETING OF THE AMERICAN-ASSOCIATION-FOR-CANCER-RESEARCH; DENVER, CA, USA; APRIL 18 -22, 2009

## Description

The present invention is in the field of immunotherapy and relates to methods for determining the efficacy of certain immunotherapy treatments. The methods of the invention include measuring special biomarker at some time following the initiation of immunotherapy treatment to evaluate the clinical outcome of the said treatment. The invention thus has applications to the field of medicine.

Traditional vaccination techniques involving the introduction into an animal system of an antigen (e.g. peptides, proteins) which can induce an immune response, and thereby protect said animal against infection for example, have been known for many years. These techniques have further included the development of both live and inactivated vaccines. Live vaccines are typically attenuated nonpathogenic versions of an infectious agent that are capable of priming an immune response directed against a pathogenic version of the infectious agent.

In recent years there have been advances in the development of recombinant vaccines, especially recombinant live vaccines, in which foreign antigens of interest are encoded and expressed from a vector. Among them, vectors based on recombinant viruses have shown great promise and play an important role in the development of new vaccines. Many viruses have been investigated for their ability to express proteins from foreign pathogens or tumor tissue, and to induce specific immunological responses against these antigens *in vivo.* Generally, these gene-based vaccines can stimulate potent humoral and cellular immune responses and viral vectors may be an effective strategy for both the delivery of antigen-encoding genes and the facilitation and enhancement of antigen presentation. In order to be utilized as a vaccine carrier, the ideal viral vector should be safe and enable efficient presentation of required pathogen-specific antigens to the immune system. Furthermore, the vector system must meet criteria that enable its production on a large-scale basis. Several viral vaccine vectors have thus emerged to date, all of them having relative advantages and limits depending on the proposed application (for a review on recombinant viral vaccines see for example Harrop and Carroll, 2006, Front Biosci., 11, 804-817 ; Yokoyama et al., 1997, J Vet Med Sci., 59, 311-322). The use of the said recombinant vaccines is commonly named targeted immunotherapy or antigen specific and active immunotherapy.

Following the observation in the early 1990's that plasmid DNA vectors could directly transfect animal cells *in vivo,* significant research efforts have also been undertaken to develop immunotherapy techniques based upon the use of DNA plasmids to induce immune response, by direct introduction into animals of DNA which encodes for antigens. Such techniques which are widely referred as DNA vaccination, or DNA immunotherapy, have now been used to elicit protective immune responses in large number of disease models. For a review on DNA vaccines, see Reyes-Sandoval and Ertl, 2001 (Current Molecular Medicine, 1, 217-243).

A general problem in immunotherapy field however has been the identification of a means of inducing a sufficiently strong immune response in treated individuals to protect against infection and disease.

Therefore there has been for example major effort in recent years, to discover new drug compounds that act by stimulating certain key aspects of the immune system which will serve to increase the immune response induced by immunotherapies. Most of these compounds, referred as immune response modifiers (IRMs) or adjuvants, appear to act through basic immune system mechanisms via Toll-like receptors (TLRs) to induce various important cytokines biosynthesis (e.g., interferons, interleukins, tumor necrosis factor, etc. see for example Schiller et al., 2006, Exp Dermatol., 15, 331-341). Such compounds have been shown to stimulate a rapid release of certain dendritic cell, monocyte/macrophage-derived cytokines and are also capable of stimulating B cells to secrete antibodies which play an important role in the antiviral and antitumor activities of IRM compounds.

Alternatively, immunotherapy strategies have been proposed, most of them being based on a prime-boost vaccination regimen. According to these "prime-boost" immunotherapy protocols, the immune system is first induced by administering to the patient a priming composition and then boosted by administration of a boosting second composition (see for example EP1411974 or US20030191076).

Moreover, it has been shown in the health care context that one treatment can be effective only in specific group of patients.

For example, WO 2007/015171 describes the treatment of non-small cell lung cancer with a liposomic formulation comprising MUC1 and IL-2 and reports that if a patient upon treatment with such a composition showed a positive MUC1 specific T-cell proliferative response, such responders had a significantly increased medium survival time compared to non-responders.

It is thus desirable to provide to physicians tools and methods that will enable them to tailor optimal personalized patient therapies, i.e. to prescribe the right therapy to the right patient at right time, to provide a higher treatment success rate, to monitor the response to the treatment, to increase drug efficacy and safety, to eliminate the unnecessary treatment of patients for whom therapy is not appropriate, to spare the patient unnecessary toxicity and side effects, to reduce the cost to patients and insurers of unnecessary or dangerous ineffective medication, and to improve patient quality of life, eventually making cancer a managed disease, with follow up assays as appropriate.

With these regards, literature proposes various tools and methods, such as for example :
- Pharmacogenetics, which consist in the study of individual response to drugs as a function of genetic differences. These responses relate to how a drug functions in any given individual, how it is metabolized, its toxicity and dosage requirements. With the human genome project, pharmacogenetics has expanded into pharmacogenomics. Pharmacogenomics goes beyond pharmacogenetics, with the potential to find uses from drug discovery and development, target discovery and validation, and clinical trials;
- Metabolomics can also be applied to the field of predictive medicine. Unlike pharmacogenetics, which is limited to genetic factors, pharmaco-metabolomics is able to predict an individual's response to a drug based not only on genetic factors, but also non-genetic factors, such as other drugs in the patient's body, the patient's current state of health, etc.
- The role of biomarkers is becoming increasingly important in the clinical development of therapeutics. A biomarker can be an indicator of normal biological processes, disease processes, or pharmacological responses to therapeutic intervention. Their role ranges from, stratifying the patient population in helping to identify responders versus non-responders to determining the efficacy of the therapeutic. Biomarkers can be a valuable tool in making better decisions that will reduce the cost for drug development and enable therapies to reach the right patient population faster. The invention provides materials and methods as described herein below for assessing the efficacy of a treatment involving the administration of an immunogenic composition to a patient (i.e. immunotherapy treatment) using biological markers (biomarkers) that have been determined to be substantially reliable signature which correlates with the desired immune response.

Thus, the present invention relates to
an ex-vivo method for assessing the efficacy of a treatment in a patient suffering of cancer with an immunogenic composition, wherein the testing method comprises the step of:
measuring levels of interferon γ in a blood, plasma or serum sample obtained from said patient after administration of the said immunogenic composition to said patient, wherein the time between the initiation of said treatment comprising administration of said immunogenic composition and interferon γ measurements is from about 4 to about 8 weeks; and
wherein levels of interferon γ above about 4 pg/ml indicate that the patient is indicative of a successful clinical outcome for the treatment, and wherein said immunogenic composition contains at least one recombinant viral vector expressing in vivo all or part of MUC1 antigen and wherein said recombinant viral vector further expresses IL-2.

The ability to predict the clinical outcome of a treatment as described above, soon after its initiation, will enable clinicians and patients to identify ineffective therapy, make informed decisions regarding the course of treatment, including whether to abandon or to allow alternate therapy implementation.

As used herein throughout the entire application, the terms "a" and "an" are used in the sense that they mean "at least one", "at least a first", "one or more" or "a plurality" of the referenced compounds or steps, unless the context dictates otherwise. For example, the term "a cell" includes a plurality of cells including a mixture thereof. More specifically, "at least one" and "one or more" means a number which is one or greater than one, with a special preference for one, two or three.

The term "and/or" wherever used herein includes the meaning of "and", "or" and "all or any other combination of the elements connected by said term".

The term "about" or "approximately" as used herein means within 20%, preferably within 10%, and more preferably within 5%.

The terms "patient", "subject" refer to a vertebrate, particularly a member of the mammalian species and includes, but is not limited to, domestic animals, sport animals, primates including humans.

As used herein, the term "treatment" or "treating" relates to therapy.

An "effective amount" or a "sufficient amount" of an active compound is an amount sufficient to effect beneficial or desired results, including clinical results. An effective amount can be administered in one or more administrations. A "therapeutically effective amount" is an amount to effect beneficial clinical results.

According to the invention, the term "assessing" should be understood as "monitoring, modifying or adjusting" a treatment involving the administration of an immunogenic composition to a patient as defined herein above.

In certain aspects, the method can further include measuring a patient's levels of interferon γ prior to immunotherapy treatment.

The time between the initiation of immunotherapy treatment and interferon γ measurements is from about 4 weeks to about 8 weeks. In a preferred embodiment of the invention, the time interval is about 5 weeks. Similarly, additional measurements (i.e., a third, fourth, fifth, etc. measurement) may be taken at similar time intervals following the second measurement.

According to a,special embodiment of the Invention, the "immunotherapy treatment" consists of at least one administration of said immunogenic composition to said patient. According to a special embodiment, the "immunotherapy treatment" consists in successive administrations of said, immunogenic composition to said patient, more specifically it consists in weekly administration during at least 2 weeks, preferably during at least 6 weeks.

The method includes determining the levels of interferon γ in a patient following administration of said immunogenic compositon to said patient; comparing said levels to a cut-off value; and assessing the efficacy of immunotherapy treatment based on the levels of interferon γ compared to the cut-off value.

The cut-off value and/or limit of detection are about 4 pg/ml (e.g. 4.6 pg/ml in plasma), preferably measured by Multi-analyte plasma protein profiling using the Luminex® system (see Example 1). The skilled man using another test/method (e.g. Elisa) will have no difficulty in determining for one specific test the equivalent of said cut-off value and/or limit of detection of about 4 pg/ml measured by Multi-analyte plasma protein profiling using the Luminex® system. According to special embodiments, the said cut-off value according to the invention can be defined as a cut-off value equivalent to cut-off value measured by Multi-analyte plasma protein profiling using the Luminex® system. By a "cut-off value equivalent to cut-off value measured by Multi-analytes plasma protein profiling using the Luminex® system" is meant a cut-off value that identifies the same patients as the one identified by cut-off value measured by Multi-analytes plasma protein profiling using the Luminex® system.

According to a special embodiment, the Invention concerns a method described herein above, wherein said treatment with said immunogenic composition comprises administering one or more doses of said immunogenic composition to said subject.

According to an alternate embodiment of the invention, the method of the invention further comprises an initial step consisting in measuring the interferon γ levels in the body of the patient before administration of the immunogenic composition.

According to the present invention, the level of interferon γ is measured in a blood, plasma or serum sample obtained from the patient.

Methods of obtaining blood or serum are well-known in the art are not part of the invention.

In addition, numerous methods for detecting and quantifying polypeptides, in particular interferon γ, are known. Such methods include but are not limited to antibody-based methods, more specifically monoclonal antibodies-based methods. The particular methods of detecting and quantifying interferon γ are not important to the invention. For example the materials and methods of the present invention may be used with Luminex technology (Luminex Corporation, Austin, Tex.) or enzyme-linked immunosorbant assays (ELISA, numerous ELISA kits are commercially available e.g. by CliniScience, Diaclone, Biosource).

As used herein, the terms "immunogenic composition" "vaccine composition", "vaccine" or similar terms can be used interchangeably.

Said immunogenic composition contains at least one recombinant viral vector expressing in *vivo* all or part of MUC1 antigen and wherein said recombinant viral vector further expresses IL-2.

According to a futher embodiment, the immunogenic composition also comprises at least one immune response modifier. Examples of such immune response modifiers (IRMs), include the CpG oligonucleotides (see US 6,194,388; US2006094683; WO 2004039829 for example), lipopolysaccharides, polyinosic:polycytidylic acid complexes (Kadowaki, et al., 2001, J. Immunol. 166, 2291-2295), and polypeptides and proteins known to induce cytokine production from dendritic cells and/or monocyte/macrophages. Other examples of such immune response modifiers (IRMs) are small organic molecule such as imidazoquinolinamines, imidazopyridine amines, 6,7-fused cycloalkylimidazopyridine amines, imidazonaphthyridine amines, oxazoloquinoline amines, thiazoloquinoline amines and 1,2-bridged imidazoquinoline amines (see for example US 4,689,338; US 5,389,640; US 6,110,929; and US 6,331,539).

If needed, the nucleic acid molecule in use in the invention may be optimized for providing high level expression of the MUC1 antigen in a particular host cell or organism, e.g. a human host cell or organism. Typically, codon optimisation is performed by replacing one or more "native" codon corresponding to a codon infrequently used in the mammalian host cell by one or more codon encoding the same amino acid which is more frequently used. This can be achieved by conventional mutagenesis or by chemical synthetic techniques (e.g. resulting in a synthetic nucleic acid). It is not necessary to replace all native codons corresponding to infrequently used codons since increased expression can be achieved even with partial replacement. Moreover, some deviations from strict adherence to optimised codon usage may be made to accommodate the introduction of restriction site(s).

As used herein, the term "recombinant vector" refers to viral vectors.

Particularly important in the context of the invention are vectors for use in gene therapy (i.e. which are capable of delivering the nucleic acid to a host organism) as well as expression vectors for use in various expression systems.

Suitable viral vectors may be derived from a variety of different viruses (e.g. retrovirus, adenovirus, AAV, poxvirus, herpes virus, measle virus, foamy virus and the like). As used herein, the term "viral vector" encompasses vector DNA/RNA as well as viral particles generated thereof. Viral vectors can be replication-competent, or can be genetically disabled so as to be replication-defective or replication-impaired. The term "replication-competent" as used herein encompasses replication-selective and conditionally-replicative viral vectors which are engineered to replicate better or selectively in specific host cells (e.g. tumoral cells).

In one aspect, the recombinant vector in use in the invention is a recombinant adenoviral vector (for a review, see "Adenoviral vectors for gene therapy", 2002, Ed D. Curiel and J. Douglas, Academic Press). It can be derived from a variety of human or animal sources and any serotype can be employed from the adenovirus serotypes 1 through 51. Particularly preferred are human adenoviruses 2 (Ad2), 5 (Ad5), 6 (Ad6), 11 (Ad11), 24 (Ad24) and 35 (Ad35). Such adenovirus are available from the American Type Culture Collection (ATCC, Rockville, Md.), and have been the subject of numerous publications describing their sequence, organization and methods of producing, allowing the artisan to apply them (see for example US 6,133,028; US 6,110,735; WO 02/40665; WO 00/50573; EP 1016711; Vogels et al., 2003, J. Virol. 77, 8263-8271).

The adenoviral vector in use in the present invention can be replication-competent. Numerous examples of replication-competent adenoviral vectors are readily available to those skill in the art (see, for example, Hernandez-Alcoceba et al., 2000, Human Gene Ther. 11, 2009-2024;'Nemunaitis et al., 2001, Gene Ther. 8, 746-759; Alemany et al., 2000, Nature Biotechnology 18, 723-727). For example, they can be engineered from a wild-type adenovirus genome by deletion in the E1A CR2 domain (see for example WO00/24408) and/or by replacement of the native E1 and/or E4 promoters with tissue, tumor or cell status-specific promoters (see for example US 5,998,205, WO99/25860, US 5,698,443, WO00/46355, WO00/15820 and WO01/36650).

Alternatively, the adenoviral vector in use in the invention is replication-defective (see for example WO94/28152; Lusky et al., 1998, J. Virol 72, 2022-2032). Preferred replication-defective adenoviral vectors are E1-defective (see for example US 6,136,594 and US 6,013,638), with an E1 deletion extending from approximately positions 459 to 3328 or from approximately positions 459 to 3510 (by reference to the sequence of the human adenovirus type 5 disclosed in the GeneBank under the accession number M 73260 and in Chroboczek et al., 1992, Virol. 186, 280-285). The cloning capacity can further be improved by deleting additional portion(s) of the adenoviral genome (all or part of the non essential E3 region or of other essential E2, E4 regions). Insertion of a nucleic acid in any location of the adenoviral vector can be performed through homologous recombination as described in Chartier et al. (1996, J. Virol. 70, 4805-4810). For example, the nucleic acid encoding the HPV-16 E6 polypeptide can be inserted in replacement of the E1 region and the nucleic acid encoding the HPV-16 E7 polypeptide in replacement of the E3 region or vice *versa*.

In another and preferred aspect, the vector in use in the invention is a pcxviral vector (see for example Cox et al. in "Viruses in Human Gene Therapy" Ed J. M. Hos, Carolina Academic Press). According to another preferred embodiment it is selected in the group consisting of vaccinia virus, suitable vaccinia viruses include without limitation the Copenhagen strain (Goebel et al., 1990, Virol. 179, 247-266 and 517-563; Johnson et al., 1993, Virol. 196, 381-401), the Wyeth strain and the highly attenuated attenuated virus derived thereof including MVA (for review see Mayr, A., et al., 1975, Infection 3, 6-14) and derivates thereof (such as MVA vaccinia strain 575 (ECACC V00120707 - US 6,913,752), NYVAC (see WO 92/15672 - Tartaglia et al., 1992, Virology, 188, 217-232). Determination of the complete sequence of the MVA genome and comparison with the Copenhagen W genome has allowed the precise identification of the seven deletions (I to VII) which occurred in the MVA genome (Antoine et al., 1998, Virology 244, 365-396), any of which can be used to insert the antigen-encoding nucleic acid. The vector may also be obtained from any other member of the poxviridae, in particular fowlpox (e.g. TROVAC, see Paoletti et al, 1995, Dev Biol Stand., 84, 159-163); canarypox (e.g. ALVAC, WO 95/27780, Paoletti et al, 1995, Dev Biol Stand., 84, 159-163); pigeonpox; swinepox and the like. By way of example, persons skilled in the art may refer to WO 92 15672 (incorporated by reference) which describes the production of expression vectors based on poxviruses capable of expressing such heterologous nucleotide sequence, especially nucleotide sequence encoding antigen.

The basic technique for inserting the nucleic acid and associated regulatory elements required for expression in a poxviral genome is described in numerous documents accessible to the man skilled in the art (Paul et al., 2002, Cancer gene Ther. 9, 470-477; Piccini et al., 1987, Methods of Enzymology 153, 545-563 ; US 4,769,330 ; US 4,772,848 ; US 4,603,112 ; US 5,100,587 and US 5,179,993). Usually, one proceed through homologous recombination between overlapping sequences (i.e. desired insertion site) present both in the viral genome and a plasmid carrying the nucleic acid to insert.

The nucleic acid encoding the MUC1 antigen is preferably inserted in a nonessential locus of the poxviral genome, in order that the recombinant poxvirus remains viable and infectious. Nonessential regions are non-coding intergenic regions or any gene for which inactivation or deletion does not significantly impair viral growth, replication or infection. One may also envisage insertion in an essential viral locus provided that the defective function is supplied *in trans* during production of viral particles, for example by using an helper cell line carrying the complementing sequences corresponding to those deleted in the poxviral genome.

When using the Copenhagen vaccinia virus, the antigen-encoding nucleic acid is preferably inserted in the thymidine kinase gene (tk) (Hruby et al., 1983, Proc. Natl. Acad. Sci USA 80, 3411-3415 ; Weir et al., 1983, J. Virol. 46, 530-537). However, other insertion sites are also appropriate, e.g. in the hemagglutinin gene (Guo et al., 1989, J. Virol. 63, 4189-4198), in the K1L locus, in the u gene (Zhou et al., 1990, J. Gen. Virol. 71, 2185-2190) or at the left end of the vaccinia virus genome where a variety of spontaneous or engineered deletions have been reported in the literature (Altenburger et al., 1989, Archives Virol. 105, 15-27 ; Moss et al. 1981, J. Virol. 40, 387-395 ; Panicali et al., 1981, J. Virol. 37, 1000-1010 ; Perkus et al, 1989, J. Virol. 63, 3829-3836 ; Perkus et al, 1990, Virol. 179, 276-286 ; Perkus et al, 1991, Virol. 180, 406-410).

When using MVA, the antigen-encoding nucleic acid can be inserted in any one of the identified deletions I to VII as well as in the D4R locus, but insertion in deletion II or III is preferred (Meyer et al., 1991, J. Gen. Virol. 72, 1031-1038 ; Sutter et al., 1994, Vaccine 12, 1032-1040).

When using fowlpox virus, although insertion within the thymidine kinase gene may be considered, the antigen-encoding nucleic acid is preferably introduced in the intergenic region situated between ORFs 7 and 9 (see for example EP 314 569 and US 5,180,675).

According to a special embodiment, said recombinant viral vector is a recombinant adenoviral vector.

According to another special embodiment, said recombinant viral vector is a recombinant vaccinia vector.

According to one preferred embodiment, said recombinant vaccinia vector is a recombinant MVA vector.

Preferably, the MUC1 antigen-encoding nucleic acid in use in the invention is in a form suitable for its expression in a host cell or organism, which means that the nucleic acid sequence encoding the MUC1 antigen are placed under the control of one or more regulatory sequences necessary for its expression in the host cell or organism. As used herein, the term "regulatory sequence" refers to any sequence that allows, contributes or modulates the expression of a nucleic acid in a given host cell, including replication, duplication, transcription, splicing, translation, stability and/or transport of the nucleic acid or one of its derivative (i.e. mRNA) into the host cell. It will be appreciated by those skilled in the art that the choice of the regulatory sequences can depend on factors such as the host cell, the vector and the level of expression desired. The nucleic acid encoding the MUC1 antigen is operatively linked to a gene expression sequence which directs the expression of the MUC1 antigen nucleic acid within a eukaryotic cell. The gene expression sequence is any regulatory nucleotide sequence, such as a promoter sequence or promoter-enhancer combination, which facilitates the efficient transcription and translation of the MUC1 antigen nucleic acid to which it is operatively linked. The gene expression sequence may, for example, be a mammalian or viral promoter, such as a constitutive or inducible promoter. Constitutive mammalian promoters include, but are not limited to, the promoters for the following genes: hypoxanthine phosphoribosyl transferase (HPRT), adenosine deaminase, pyruvate kinase, b-actin promoter and other constitutive promoters. Exemplary viral promoters which function constitutively in eukaryotic cells include, for example, promoters from the cytomegalovirus (CMV), simian virus (e.g., SV40), papilloma virus, adenovirus, human immunodeficiency virus (HIV), Rous sarcoma virus, cytomegalovirus, the long terminal repeats (LTR) of Moloney leukemia virus and other retroviruses, and the thymidine kinase promoter of herpes simplex virus. Other constitutive promoters are known to those of ordinary skill in the art.

The promoters useful as gene expression sequences of the invention also include inducible promoters. Inducible promoters are expressed in the presence of an inducing agent. For example, the metallothionein promoter is induced to promote transcription and translation in the presence of certain metal ions. Other inducible promoters are known to those of ordinary skill in the art. In general, the gene expression sequence shall include, as necessary, 5' non-transcribing and 5' non-translating sequences involved with the initiation of transcription and translation, respectively, such as a TATA box, capping sequence, CAAT sequence, and the like. Especially, such 5' non-transcribing sequences will include a promoter region which includes a promoter sequence for transcriptional control of the operably joined antigen nucleic acid. The gene expression sequences optionally include enhancer sequences or upstream activator sequences as desired. Preferred promoters for use in a poxviral vector (see below) include without limitation vaccinia promoters 7.5K, H5R, TK, p28, p11 and K1L, chimeric promoters between early and late poxviral promoters as well as synthetic promoters such as those described in Chakrabarti et al. (1997, Biotechniques 23, 1094-1097), Hammond et al. (1997, J. Virological Methods 66, 135-138) and Kumar and Boyle (1990, Virology 179, 151-158).

The promoter is of special importance and the present invention encompasses the use of constitutive promoters which direct expression of the nucleic acid in many types of host cells and those which direct expression only in certain host cells or in response to specific events or exogenous factors (e.g. by temperature, nutrient additive, hormone or other ligand). Suitable promoters are widely described in literature and one may cite more specifically viral promoters such as RSV, SV40, CMV and MLP promoters. Preferred promoters for use in a poxviral vector include without limitation vaccinia promoters 7.5K, H5R, TK, p28, p11 and K1L, chimeric promoters between early and late poxviral promoters as well as synthetic promoters such as those described in Chakrabarti et al. (1997, Biotechniques 23, 1094-1097), Hammond et al. (1997, J. Virological Methods 66, 135-138) and Kumar and Boyle (1990, Virology 179, 151-158).

Those skilled in the art will appreciate that the regulatory elements controlling the expression of the nucleic acid molecule encoding MUC1 antigen may further comprise additional elements for proper initiation, regulation and/or termination of transcription (e.g. polyA transcription termination sequences), mRNA transport (e.g. nuclear localization signal sequences), processing (e.g. splicing signals), and stability (e.g. introns and non-coding 5' and 3' sequences), translation (e.g. peptide signal, propeptide, tripartite leader sequences, ribosome binding sites, Shine-Dalgamo sequences, etc.) into the host cell or organism.

The recombinant viral vector in use in the present invention further comprises a nucleic acid encoding IL-2. It can comprise further nucleic acids encoding other cytokines. Suitable cytokines include without limitation interleukins (e.g. IL-7, IL-15, IL-18, IL-21) and interferons (e.g. IFNγ, INFα). Such a further cytokine-expressing nucleic acid may be carried by the recombinant viral vector encoding MUC 1 and IL-2 or by an independent recombinant vector which can be of the same or a different origin.

Infectious viral particles comprising the above-described recombinant viral vector can be produced by routine process. An exemplary process comprises the steps of:
a. introducing the viral vector into a suitable cell line,
b. culturing said cell line under suitable conditions so as to allow the production of said infectious viral particle,
c. recovering the produced infectious viral particle from the culture of said cell line, and
d. optionally purifying said recovered infectious viral particle.

Cells appropriate for propagating adenoviral vectors are for example 293 cells, PERC6 cells, HER96 cells, or cells as disclosed in WO 94/28152, WO 97/00326, US 6,127,175.

Cells appropriate for propagating poxvirus vectors are avian cells, and most preferably primary chicken embryo fibroblasts (CEF) prepared from chicken embryos obtained from fertilized eggs.

The infectious viral particles may be recovered from the culture supernatant or from the cells after lysis (e.g. by chemical means, freezing/thawing, osmotic shock, mecanic shock, sonication and the like). The viral particles can be isolated by consecutive rounds of plaque purification and then purified using the techniques of the art (chromatographic methods, ultracentrifugation on caesium chloride or sucrose gradient).

According to another embodiment, the methods of the invention may be combined with other methods for predicting the efficacy of treatment more specifically for predicting the efficacy of immunotherapy treatments. For example, levels of biomarkers such as levels of activated NK cells (see patent application claiming priority of EP 08305876.8) or levels of sICAM-1 (see patent application claiming priority of EP 09305032.6).

If desired, the administration of the immunogenic composition can be carried out in conjunction with one or more conventional therapeutic modalities (e.g. radiation, chemotherapy and/or surgery). The use of multiple therapeutic approaches provides the selected patient with a broader based intervention. In one embodiment, the administration of the immunogenic composition can be preceded or followed by a surgical intervention. In another embodiment, it can be preceded or followed by radiotherapy (e.g. gamma radiation). Those skilled in the art can readily formulate appropriate radiation therapy protocols and parameters which can be used (see for example Perez and Brady, 1992, Principles and Practice of Radiation Oncology, 2nd Ed. JB Lippincott Co; using appropriate adaptations and modifications as will be readily apparent to those skilled in the field). In still another embodiment, the administration of the immunogenic composition is associated to chemotherapy with one or more drugs (e.g. drugs which are conventionally used for treating or preventing viral infections, virus-associated pathologic conditions, cancer, and the like).

Thus, also disclosed is a method for improving the treatment of a cancer patient which is undergoing chemotherapeutic treatment with a chemotherapeutic agent, said method comprising the following steps :
- administering to cancer patients one or more doses of an immunogenic composition as described herein above and one or more doses of a chemotherapeutic agent.
- measuring an interferon γ level in a blood, plasma or serum sample of said patient following at least one of the said administration of said immunogenic composition as described herein above;
- wherein levels of interferon γ above about 4 pg/ml (e.g. 4.6 pg/ml) indicates that the subject is indicative of a successful clinical outcome for the treatment, i.e., the increase in survival rate.

The administration of said chemotherapeutic agent can be done before administration of said immunogenic composition, after administration of said immunogenic composition, or concomitantly with administration of said immunogenic composition.

In another embodiment, in the method of the invention the treatment with the immunogenic composition which the cancer patient receives is carried out according to a prime boost therapeutic modality which comprises sequential administration of one or more primer composition (s) and one or more booster composition (s). Typically, the priming and the boosting compositions use different vehicles which comprise or encode at least an antigenic domain in common. The priming composition is initially administered to the host organism and the boosting composition is subsequently administered to the same host organism after a period varying from one day to twelve months. Such a prime boost modality may comprise one to ten sequential administrations of the priming composition followed by one to ten sequentiel administrations of the boosting composition. Desirably, injection intervals are a matter of one week to six months. Moreover, the priming and boosting compositions can be administered at the same site or at alternative sites by the same route or by different routes of administration.

According to a special embodiment, said cancer is for example breast cancer, colon cancer, kidney cancer, rectal cancer, lung cancer, cancer of the head and neck, renal cancer, malignant melanoma, laryngeal cancer, ovarian cancer, cervical cancer, prostate cancer, non Small cell Lung Cancer (NSCLC), haematological cancers, gastric cancers, myeloma.

According to a preferred embodiment, said cancer is non Small cell Lung Cancer (NSCLC).

According to one special embodiment, an immune response can be observed in the treated cancer patient directed towards MUC1 antigen. According to one embodiment, said "immune response" in said patient population is directed towards distinct antigens.

According to another special embodiment, said "immune response" in said cancer patient is T cell immune response, and preferably CD8+ (Cytotoxic T Lymphocytes) immune response. According to another special embodiment, said "immune response" in said patient is a non specific immune response. According to another special embodiment, said "immune response" in said patient is a stimulation of the innate immune response.

The ability to induce or stimulate an immune response upon administration in an animal or human organism can be evaluated either *in vitro* or *in vivo* using a variety of assays which are standard in the art. For a general description of techniques available to evaluate the onset and activation of an immune response, see for example Coligan et al. (1992 and 1994, Current Protocols in Immunology ; ed J Wiley & Sons Inc, National Institute of Health). Measurement of cellular immunity can be performed by measurement of cytokine profiles secreted by activated effector cells including those derived from CD4+ and CD8+ T-cells (e.g. quantification of IL-10 or IFN gamma-producing cells by ELIspot), by determination of the activation status of immune effector cells (e.g. T cell proliferation assays by a classical [³H] thymidine uptake), by assaying for antigenspecific T lymphocytes in a sensitized subject (e.g. peptidespecific lysis in a cytotoxicity assay) or by detection of antigen specific T cells by fluorescent MHC and/or peptide multimers (e.g. tetramers). The ability to stimulate a humoral response may be determined by antibody binding and/or competition in binding (see for example Harlow, 1989, Antibodies, Cold Spring Harbor Press). The method of the invention can also be further validated in animal models challenged with an appropriate tumor-inducing agent (e.g. MUC1-expressing RMA cells) to determine anti-tumor activity, reflecting an induction or an enhancement of an anti-antigen immune response.

Thus, also described is a method for extending the survival of a cancer patient treated by administering an immunogenic composition as defined herein above, said method comprising the following step:
- administering to a patient one or more doses of an immunogenic composition as defined herein above,
- measuring an interferon γ level of said patient following at least one of the said administration of said immunogenic composition as described herein above.

Also disclosed is the use of interferon γ as a biomarker for predicting whether a cancer patient treated by administration of an immunogenic composition as defined herein above is or is not susceptible to developing a therapeutic response, preferably a therapeutic immune response as a follow up to the said administration of an immunogenic composition.

Further disclosed is the use of interferon γ as a biomarker for monitoring, modifying or adjusting a treatment involving the administration of an immunogenic composition as defined herein above to a cancer patient.

Also disclosed is the use of the level of interferon γ as a biomarker for monitoring, modifying of adjusting a treatment involving the administration of an immunogenic composition as defined herein above to a cancer patient, wherein levels of a interferon γ measured after said administration as defined herein above 4 pg/ml (e.g. 4.6 pg/ml) indicate that the patient is indicative of a successful clinical outcome for the treatment, i.e., the increase in survival rate.

The present invention also relates to the use of a kit for testing in an ex-vivo method according to the present invention whether a patient suffering of cancer is responding therapeutically to a method of treatment with an immunogenic composition, wherein the kit comprises:
(a) antibodies for determining the level of interferon γ in said blood, plasma or serum sample from the patient; and
(b) instructions for interpreting the data obtained saying that levels of interferon γ above about 4 pg/ml indicate that the subject is indicative of a successful clinical outcome for the treatment in accordance with the method of the present invention;
wherein said immunogenic composition contains at least one recombinant viral vector expressing in vivo all or part of MUC1 antigen and wherein said recombinant viral vector further expresses IL-2; and
wherein the time between the initiation of said treatment comprising administration of said immunogenic composition and interferon γ measurements is from about 4 to about 8 weeks.

The invention thus also discloses kits which include parts for practicing the methods described herein and that will be apparent from the examples provided herein. The kit of parts, or kits, may include reagents for collecting and or measuring serum levels of interferon gamma. Such reagents are antibodies. The kits may further include equipment for collecting and/or processing biological samples. The kits also contain instructions for use, cut-off values and/or instructions for their determination, and instructions for interpreting the data obtained from the use of the kits.

### EXAMPLES

### Figures :

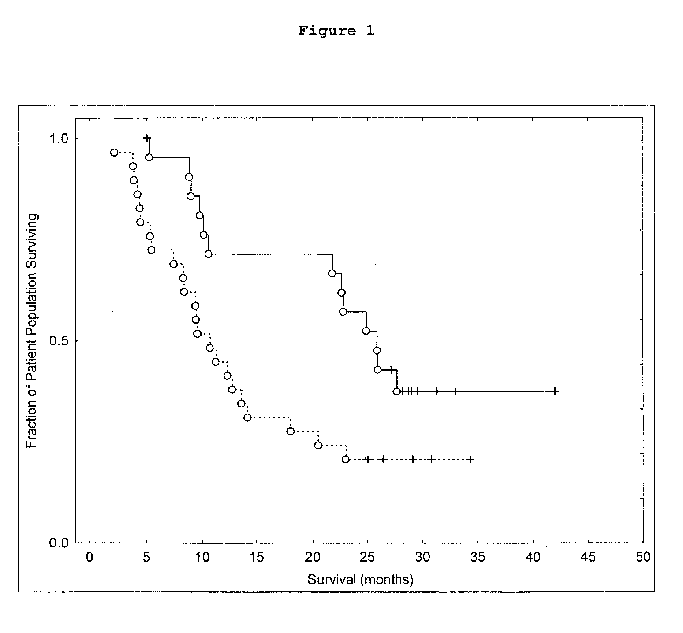

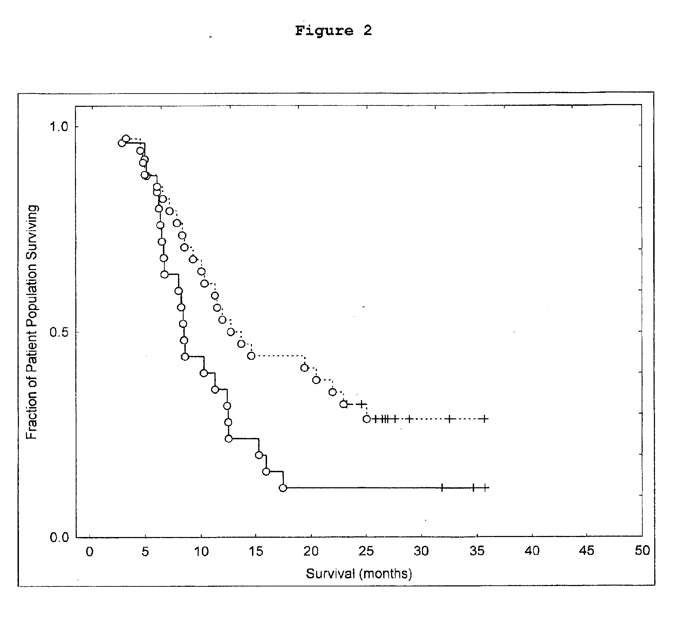

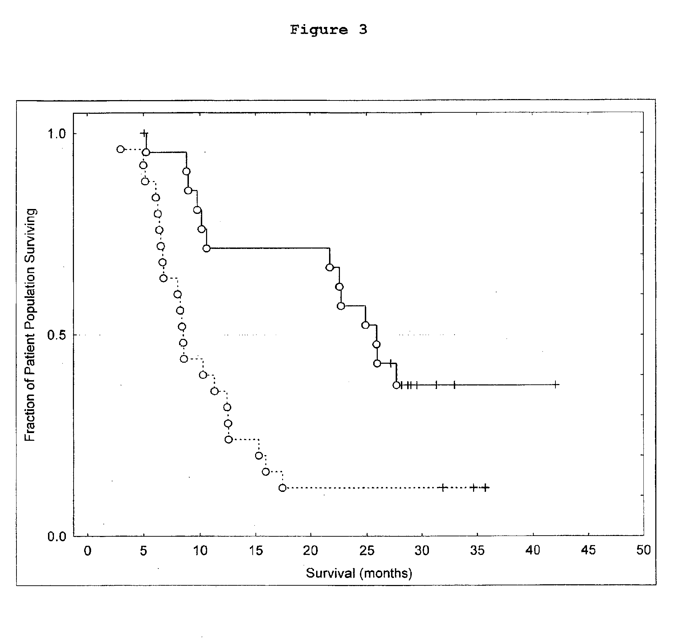

**Figure 1****: Survival curves describing vaccine immunotherapy in lung cancer: patients with or without detectable interferon γ at Day 43**
   - Group 1 : Therapeutic vaccine (i.e. immunogenic composition) + chemotherapy in patients with detectable interferon γ at Day 43. Detectable as defined as ≥ Limit of Detection which is 4.6 pg/ml. 22 patients. Median Survival 25.4 months.
   - Group 2 : Therapeutic vaccine (i.e. immunogenic composition) + chemotherapy in patients with no detectable interferon γ. Not detectable as defined as < 4.6 pg/ml sCD54 in peripheral blood. 29 patients. Median Survival 10.14 months
   Significant difference, by log rank: p = 0.019
   **O** Complete + Censored
**Figure 2****: Survival curves describing chemotherapy in lung cancer: patients with or without detectable (≥ 4.6 pg/ml) interferon γ**
   - Group 1 : Chemotherapy alone (without therapeutic vaccine) in patients with detectable interferon γ at Day 43. Detectable as defined as > 4.6 pg/ml in peripheral blood. 25 patients. Median Survival 8.5 months.
   - Group 2 : Chemotherapy alone (without therapeutic vaccine) in patients with no detectable interferon γ at Day 43. Not detectable as defined as < 4.6 pg/ml IFNg in peripheral blood. 34 patients. Median Survival 12.8 months.
   Significant difference, by log rank: p = 0.047
   **O** Complete **+** Censored
**Figure 3****: Survival curves describing chemotherapy in lung cancer: patients with ≥ 4.6 pg/ml) interferon γ**
   - Group 1 Therapeutic vaccine (i.e. immunogenic composition) + chemotherapy in patients with detectable levels of interferon y. Detectable levels as defined as ≥ 4.6 pg/ml in peripheral blood. 22 patients. Median Survival 25.4 months.
   - Group 2 : Chemotherapy alone (without TG4010 vaccine) in patients with detectable levels of interferon γ. Detectable levels as defined as ≥ 4.6 pg/ml interferon γ in peripheral blood. 25 patients. Median Survival 8.5 months.
   Significant difference, by log rank: p = 0.002
   **O** Complete + Censored

### Example 1 :

The immunogenic composition, noted vaccine TG4010, was used to treat non-small cell lung cancer (NSCLC) patients in combination with standard chemotherapy.

TG4010 is a recombinant Modified Virus Ankara (MVA) expressing both IL2 and the tumor-associated antigen MUC1.

One hundred and forty eight patients were randomized to receive:
- chemotherapy (Cisplatin 75mg/m² on d1 and Gemcitabine 1250mg/m² on day 1 and day 8 every 3 weeks for up to 6 cycles) either alone (Study Arm 2) or
- chemotherapy together with TG4010 (Study Arm 1).

Tumors were evaluated (WHO criteria) every 6 weeks. Endpoints were progression-free survival (PFS) at 6 months and overall survival with intent to treat analysis.

Blood samples were taken at day 43 (one week following the 6^{th} weekly injection) and were shipped immediately to a central immunology lab where plasma samples were aliquoted and frozen. Frozen aliquots of plasma were shipped, in batch, on dry ice, to a second central lab where interferon gamma levels were assessed.

Plasma samples were assessed for content of Interferon gamma (interferon γ) by Multi-analyte plasma protein profiling using the Luminex® system. The limit of detection has been established as 4.6 pg/ml.

Figure 1 shows that patients [Arm 1 (TG4010 + chemotherapy] with detectable interferon γ at day 43 survive lounger (median survival = 25.4 months) than do patients with no detectable interferon γ (median survival 10.14 months) when treated with both the TG4010 vaccine and chemotherapy.

The data in Figure 2 demonstrate that the positive association between detectable plasma interferon γ and overall survival is restricted to patients receiving the therapeutic vaccine. Patients in arm 2, receiving chemotherapy alone, who had detectable plasma interferon γ at Day 43 had poorer survival (8.5 months) than did patients with no detectable interferon γ at Day 43 (12.8 months).

The data in Figures 3 demonstrates that the effect of selecting patients based on the detection of interferon γ in their day 43 plasma is restricted to patients receiving the therapeutic vaccine. Figure 3 shows that patients with detectable interferon γ at Day 43 have a superior survival expectancy only if they receive the therapeutic vaccine.

## Claims

1. An ex-vivo method for assessing the efficacy of a treatment in a patient suffering of cancer with an immunogenic composition, wherein the testing method comprises the step of:
measuring levels of interferon γ in a blood, plasma or serum sample obtained from said patient after administration of the said immunogenic composition to said patient, wherein the time between the initiation of said treatment comprising administration of said immunogenic composition and interferon γ measurements is from about 4 to about 8 weeks; and
wherein levels of interferon γ above about 4 pg/ml indicate that the patient is indicative of a successful clinical outcome for the treatment, and wherein said immunogenic composition contains at least one recombinant viral vector expressing in vivo all or part of MUC1 antigen and wherein said recombinant viral vector further expresses IL-2.

2. The method of claim 1 wherein the level of interferon γ is above about 4.6 pg/ml.

3. The method of claim 1 or 2, wherein the time between the initiation of said treatment comprising administration of said immunogenic composition and interferon γ measurements is about 5 weeks.

4. The method of any one of claims 1 to 3, wherein said cancer is non Small cell Lung Cancer (NSCLC).

5. The method of any one of claims 1 to 4, wherein said levels of interferon γ is measured by Multi-analyte plasma protein profiling using the Luminex® system.

6. The method of any one of claims 1 to 5, wherein said viral vector is replication-competent.

7. The method of any one of claims 1 to 5, wherein said viral vector is replication-defective.

8. The method of claim 6 or 7, wherein said recombinant viral vector is a recombinant vaccinia virus vector.

9. The method of claim 8, wherein said recombinant vaccinia virus vector is a recombinant MVA vector.

10. The method of any one of claims 1 to 9, wherein said patient is a patient treated with a chemotherapeutic agent.

11. The method of claim 10, wherein the administration of said chemotherapeutic agent is done concomitantly with administration of said immunogenic composition.

12. The method of any one of claims 1 to 11, wherein said method further comprises an initial step consisting in measuring the interferon γ levels in a blood, plasma or serum sample obtained from said patient before said administration of said immunogenic composition.

13. Use of a kit for testing in an ex-vivo method according to claim 1 whether a patient suffering of cancer is responding therapeutically to a method of treatment with an immunogenic composition, wherein the kit comprises:
(a) antibodies for determining the level of interferon γ in said blood, plasma or serum sample from the patient; and
(b) instructions for interpreting the data obtained saying that levels of interferon γ above about 4 pg/ml indicate that the subject is indicative of a successful clinical outcome for the treatment in accordance with the method of claim 1;
wherein said immunogenic composition contains at least one recombinant viral vector expressing in vivo all or part of MUC1 antigen and wherein said recombinant viral vector further expresses IL-2; and
wherein the time between the initiation of said treatment comprising administration of said immunogenic composition and interferon γ measurements is from about 4 to about 8 weeks.

14. The use of claim 13, wherein the level of interferon γ is above about 4.6 pg/ml.

## Patentansprüche

1. *Ex vivo*-Verfahren zur Bewertung der Wirksamkeit einer Behandlung mit einer immunogenen Zusammensetzung bei einem Individuum, das an Krebs leidet, wobei das Testverfahren die folgenden Schritte umfasst:
Messen der Interferon-γ-Spiegel in einer Blut-, Plasma- oder Serumprobe, die von dem Individuum nach der Verabreichung der immunogenen Zusammensetzung an das Individuum erhalten wird,
wobei der Zeitraum zwischen dem Beginn der Behandlung, die die Verabreichung der immunogenen Zusammensetzung umfasst, und den Interferon-γ-Messungen etwa 4 bis etwa 8 Wochen ist; und
wobei Interferon-γ-Spiegel über etwa 4 pg/ml anzeigen, dass sich bei dem Individuum bei der Behandlung ein erfolgreiches klinisches Ergebnis erkennen lässt, und wobei die immunogene Zusammensetzung mindestens einen rekombinanten viralen Vektor umfasst, der *in vivo* das ganze oder einen Teil des MUC1-Antigens exprimiert, und wobei der rekombinante virale Vektor des Weiteren IL-2 exprimiert.

2. Verfahren nach Anspruch 1, wobei der Interferon-γ-Spiegel über etwa 4,6 pg/ml liegt.

3. Verfahren nach Anspruch 1 oder 2, wobei der Zeitraum zwischen dem Beginn der Behandlung, die die Verabreichung der immunogenen Zusammensetzung umfasst, und den Interferon-γ-Messungen etwa 5 Wochen ist.

4. Verfahren nach einem der Ansprüche 1 bis 3, wobei der Krebs nichtkleinzelliger Lungenkrebs (NSCLC) ist.

5. Verfahren nach einem der Ansprüche 1 bis 4, wobei die Interferon-γ-Spiegel durch multianalytische Plasmaproteinanalyse unter Verwendung des Luminex®-Systems gemessen werden.

6. Verfahren nach einem der Ansprüche 1 bis 5, wobei der virale Vektor replikationskompetent ist.

7. Verfahren nach einem der Ansprüche 1 bis 5, wobei der virale Vektor replikationsdefizient ist.

8. Verfahren nach Anspruch 6 oder 7, wobei der rekombinante virale Vektor ein rekombinanter Vacciniavirus-Vektor ist.

9. Verfahren nach Anspruch 8, wobei der rekombinante Vacciniavirus-Vektor ein rekombinanter MVA-Vektor ist.

10. Verfahren nach einem der Ansprüche 1 bis 9, wobei das Individuum ein Individuum ist, das mit einem Chemotherapeutikum behandelt wird.

11. Verfahren nach Anspruch 10, wobei die Verabreichung des Chemotherapeutikums gleichzeitig mit der Verabreichung der immunogenen Zusammensetzung erfolgt.

12. Verfahren nach einem der Ansprüche 1 bis 11, wobei das Verfahren des Weiteren einen ersten Schritt umfasst, bestehend aus dem Messen der Interferon-γ-Spiegel in einer Blut-, Plasma- oder Serumprobe, die von dem Individuum vor der Verabreichung der immunogenen Zusammensetzung erhalten wurde.

13. Verwendung eines Kits zum Testen in einem ex *vivo*-Verfahren nach Anspruch 1, ob ein Individuum, das an Krebs leidet, therapeutisch auf ein Behandlungsverfahren mit einer immunogenen Zusammensetzung anspricht, wobei der Kit umfasst:
(a) Antikörper zum Bestimmen des Interferon-γ-Spiegels in der Blut-, Plasma- oder Serumprobe des Individuums; und
(b) Anweisungen zum Auswerten der erhaltenen Daten, die besagen, dass Interferon-γ-Spiegel über etwa 4 pg/ml anzeigen, dass sich bei dem Individuum bei der Behandlung in Übereinstimmung mit dem Verfahren nach Anspruch 1 ein erfolgreiches klinisches Ergebnis erkennen lässt;
wobei die immunogene Zusammensetzung mindestens einen rekombinanten viralen Vektor umfasst, der *in vivo* das ganze oder einen Teil des MUC1-Antigens exprimiert, und wobei der rekombinante virale Vektor des Weiteren IL-2 exprimiert; und
wobei der Zeitraum zwischen dem Beginn der Behandlung, die die Verabreichung der immunogenen Zusammensetzung umfasst, und den Interferon-γ-Messungen etwa 4 bis etwa 8 Wochen ist;

14. Verwendung nach Anspruch 13, wobei der Interferon-γ-Spiegel über etwa 4,6 pg/ml ist.

## Revendications

1. Méthode ex-vivo pour évaluer l'efficacité d'un traitement avec une composition immunogène chez un patient souffrant d'un cancer, la méthode d'analyse comprenant les étapes consistant à :
mesurer des taux de l'interféron γ dans un échantillon de sang, de plasma ou de sérum obtenu auprès dudit patient après l'administration de ladite composition immunogène audit patient, dans laquelle le temps entre l'initiation dudit traitement comprenant l'administration de ladite composition immunogène et les mesures de l'interféron γ est d'environ 4 à environ 8 semaines ; et
dans laquelle des taux de l'interféron γ supérieurs à environ 4 pg/ml indiquent que le patient montre une issue clinique réussie pour le traitement, et dans laquelle ladite composition immunogène contient au moins un vecteur viral recombinant exprimant in vivo tout ou une partie de l'antigène MUC1 et dans laquelle ledit vecteur viral recombinant exprime en outre l'IL-2.

2. Méthode selon la revendication 1, dans laquelle le taux de l'interféron γ est supérieur à environ 4,6 pg/ml.

3. Méthode selon la revendication 1 ou 2, dans laquelle le temps entre l'initiation dudit traitement comprenant l'administration de ladite composition immunogène et les mesures de l'interféron est d'environ 5 semaines.

4. Méthode selon l'une quelconque des revendications 1 à 3, dans laquelle ledit cancer est un cancer du poumon non à petites cellules (NSCLC).

5. Méthode selon l'une quelconque des revendications 1 à 4, dans laquelle lesdits taux de l'interféron γ sont mesurés par un profilage de protéines plasmatiques multi-analyte en utilisant le système Luminex®.

6. Méthode selon l'une quelconque des revendications 1 à 5, dans laquelle ledit vecteur viral est compétent pour la réplication.

7. Méthode selon l'une quelconque des revendications 1 à 5, dans laquelle ledit vecteur viral est défectueux pour la réplication.

8. Méthode selon la revendication 6 ou 7, dans laquelle ledit vecteur viral recombinant est un vecteur du virus de la vaccine recombinant.

9. Méthode selon la revendication 8, dans laquelle ledit vecteur du virus de la vaccine recombinant est un vecteur MVA recombinant.

10. Méthode selon l'une quelconque des revendications 1 à 9, dans laquelle ledit patient est un patient traité par un agent chimiothérapeutique.

11. Méthode selon la revendication 10, dans laquelle l'administration dudit agent chimiothérapeutique est réalisée de manière concomitante avec l'administration de ladite composition immunogène.

12. Méthode selon l'une quelconque des revendications 1 à 11, dans laquelle ladite méthode comprend en outre une étape initiale consistant à mesurer les taux de l'interféron γ dans un échantillon de sang, de plasma ou de sérum obtenu auprès dudit patient avant ladite administration de ladite composition immunogène.

13. Utilisation d'un kit pour tester dans une méthode ex-vivo selon la revendication 1, si un patient souffrant d'un cancer répond ou non de manière thérapeutique à une méthode de traitement avec une composition immunogène, dans laquelle le kit comprend :
(a) des anticorps pour déterminer le taux de l'interféron γ dans ledit échantillon de sang, de plasma ou de sérum obtenu auprès du patient ; et
(b) les instructions d'interprétation des données obtenues indiquant que des taux de l'interféron γ supérieurs à environ 4 pg/ml indiquent que le patient montre une issue clinique réussie pour le traitement conformément à la méthode selon la revendication 1 ;
dans laquelle ladite composition immunogène contient au moins un vecteur viral recombinant exprimant in vivo tout ou une partie de l'antigène MUC1 et dans laquelle ledit vecteur viral recombinant exprime en outre l'IL-2 ; et
dans laquelle le temps entre l'initiation dudit traitement comprenant l'administration de ladite composition immunogène et les mesures de l'interféron γ est d'environ 4 à environ 8 semaines.

14. Utilisation selon la revendication 13, dans laquelle le taux de l'interféron γ est supérieur à environ 4,6 pg/ml.
